# EUROPEAN PATENT SPECIFICATION

(11) **EP 2 552 562 B1**
(45) Date of publication and mention of the grant of the patent: **08.10.2014**
(21) Application number: 11762073.2
(22) Date of filing: 29.03.2011
(51) Int. Cl.: B01D 15/18, C13K 13/00, C13B 35/06, C07C 229/12

(54) **SEPARATION PROCESS**
SEPARATIONSVERFAHREN
PROCÉDÉ DE SÉPARATION

(30) Priority: 28.05.2010 US 349307 P; 30.03.2010 US 318950 P
(43) Date of publication of application: 06.02.2013
(73) Proprietor: DuPont Nutrition Biosciences ApS, 1001 Copenhagen K (DK)
(72) Inventor: AIRAKSINEN, Jyrki, FI-02760 Espoo (FI); PAANANEN, Hannu, FI-02460 Kantvik (FI); LEWANDOWSKI, Jari, FI-02580 Siuntio (FI); LAIHO, Kari, FI-02400 Kirkkonummi (FI)
(74) Representative: Kolster Oy Ab
(86) International application number: PCT/FI2011/050262
(87) International publication number: WO 2011/121179

(56) References cited:
- WO-A1-97/45185
- US-A1- 2005 115 904
- US-A1- 2005 115 904
- US-B1- 6 224 776
- US-B1- 6 224 776
- US-B2- 6 875 349
- US-B2- 6 875 349

## Description

### FIELD OF THE INVENTION

The invention relates to a process of recovering sucrose and betaine from sugar beet derived syrups by chromatographic sequential multiprofile simulated moving bed (SMB) methods. The sugar beet derived syrup specifically used as the starting material in the invention is thick juice or concentrated raw juice having a high sucrose content, typically a sucrose content of more than 85% on the dry substance basis.

### BACKGROUND OF THE INVENTION

US 6 875 349 B2 discloses a method and a system for fractionating a sugar-beet based solution, such as molasses and vinasse, into two or more fractions by a chromatographic simulated moving bed (SMB) process, wherein the separation system comprises at least two separation profiles in the same loop. The SMB process may be continuous or sequential. It is recited that the two profiles are formed by adding at least two portions of the feed solution to the system before recovering product fractions therefrom (column 5, lines 9 to 11). It is also recited that water may be used as an eluent and that water can be added between or after the feed solution additions to ensure no overlap of the two separation profiles (column 5, lines 11 to 14). It is also recited that there can be two or three parallel operations within one separation step (column 6, lines 7 to 8). Furthermore, it is recited that the two or more profiles are moving in the entire resin bed (all columns in the loop) (column 1, lines 32 to 34), i.e. said at least two separation profiles are present in a loop formed by all columns.

Example 1 of US 6 875 349 B2 discloses two-profile sequential SMB separation of molasses in a separation system comprising two columns (with eight separate partial packed beds altogether). The fractionation is performed in an eight-step sequence with a sequence length of 38 minutes. Molasses used as the starting material contains 57.6% sucrose and 7.6% betaine on DS. The fractionation provides a sucrose fraction with a purity of 90.1% by weight and a betaine fraction with a purity of 43.1 % by weight.

Example 4 of US 6 875 349 B2 discloses three-profile sequential SMB separation of molasses in a separation system comprising three separate columns. The fractionation is performed in an eight-step sequence with a sequence length of 43 minutes. Molasses used as the starting material contains 60.4% sucrose and 5.1% betaine on DS. The fractionation provides a sucrose fraction with a purity of 92.7% by weight and a betaine fraction with a purity of 36.6% by weight.

Example 5 of US 6 875 349 B2 discloses two-profile continuous SMB separation of molasses containing 59% sucrose. A sucrose fraction having a purity of 87.8% is obtained.

US 6 224 683 B1 discloses a process of purifying (demineralizing) a beet sugar solution by a chromatographic SMB separation process for providing a purified sucrose fraction. Example 1 discloses continuous separation of a softened sucrose-containing solution containing 93% sucrose, 4% non-sucrose compounds such as salts, and 3% other organic compounds (other saccharides, betaine, amino acids etc.) with a 10-column SMB system to provide a sucrose fraction and a non-sucrose fraction. The sucrose fraction is recited to have a sucrose recovery of 99.4%, a proportion of mixed non-sucrose compounds of 20% and a sucrose concentration (Bx) of 38.

US 5 466 294 discloses a continuous chromatographic single-profile method for separating sucrose from a soft raw juice obtained from sugar beets. Example 1 discloses the separation of softened concentrated raw juice containing 88.6% sucrose and 1.2% betaine. A sucrose fraction having a sucrose purity of 97.4% and a betaine content of 0.9% is obtained. The process provides a very low separation capacity of 35 lbs dry substance per cubic feet of the resin per day, which corresponds to 23.5 kg dry substance per hour per m³ of the resin.

The above-described processes of US 6 224 683 B1 and US 5 466 294 do not recover betaine from thick juice or raw juice.

US 6 093 326 discloses a method for the fractionation of molasses by a chromatographic SMB system, wherein sucrose and betaine are recovered during a multistep separation sequence in two or more loops. Example 3 of the document discloses a process for the fractionation of beet molasses with a step comprising three recycling phases.

However, multiprofile sequential SMB separation processes for recovering sucrose and betaine from sugar beet based solutions having a high sucrose content (more than 85%) are not suggested or disclosed in the art.

### DEFINITIONS RELATING TO THE INVENTION

"A product fraction" is a fraction taken out of the chromatographic separation process and comprising product components. Product components can be selected from sucrose, betaine, raffinose and pyrrolidone carboxylic acid (PCA). There can be one or more product fractions.

"A residue fraction" or "a residual fraction" is a fraction which mainly contains components (such as salts, color compounds, organic acids, amino acids, etc.) other than the product components which are recovered. There can be one or more residue fractions. The components of the residual fraction are also referred to as "residual components".

"A recycle fraction" is a fraction, which contains incompletely separated product compounds and which generally has a lower purity or is more dilute than the product fractions. The recycle fraction is recycled back to the separation to be combined with the feed. The recycle fraction is typically used as a diluent of the feed. There may also be one or more operations before returning the recycle to the column(s); for example, the recycle fraction(s) may be concentrated by evaporation. There can be one or more recycle fractions.

"A sequence" or "a separation sequence" is a predetermined sequence of steps which are continuously repeated in a sequential chromatographic separation process, comprising all steps that are required to facilitate the separation of feed components to product fraction(s) and other fractions.

"A step" comprises one or more of a feeding phase, an elution phase and a circulation phase, and optionally a part feeding phase.

During the feeding phase, a feed solution is introduced into a predetermined partial packed bed or into predetermined partial packed beds. During the feeding phase, and/or one or more other phases, one or more product fractions and one or more residual fractions can be withdrawn.

During the elution phase, an eluent is fed into predetermined partial packed beds.

During the circulation phase, essentially no feed solution or eluent is supplied to the partial packed beds and no products are withdrawn.

During the part feeding phase, a part of the separation profile is introduced back to the separation as a substitute of eluent. The part feeding phase is present when applying the methods of WO 2010/097513 A1 and WO 2010/097511 A1 (US 2010-0212662 A1) to the multiprofile method of the invention. The whole content of the recited documents is incorporated herein by reference.

"SMB" refers to a simulated moving bed system.

In a sequential SMB system, not all the fluid streams flow continuously. These streams are: the supply of a feed solution and an eluent (or PART), circulation of the separation profile and withdrawal of the products, recycle or PARTS.

"A feed" is an amount of feed solution introduced to the separation column(s) during one sequence.

"Concentrated raw juice" and "thick juice" refer to sugar beet based syrups (a more detailed definition thereof is presented in "Description of the invention"). A sucrose extract from sugar beet is called raw juice, which is purified to produce thin juice, called after evaporation as thick juice.

"A subprofile" is a concentration profile of one component, also named as the component peak. In connection with the present invention, "a sucrose subprofile" is the concentration profile of sucrose, "a betaine subprofile" is the concentration profile of betaine, and "a residual subprofile" is the concentration profile of the residual components, including salts, color compounds, organic acids, amino acids, etc.

"A separation profile" refers to a dry substance profile formed from the dissolved substances (DS) present in the feed on account of feedingof eluent and feed solution and the flow through the resin bed, obtained by accomplishing/repeating the separation sequence.

"A part of the separation profile" equal to " PART " refers to any section of the separation profile which contains liquid and components in this section and which is used as an eluent replacement.

"Tailing" and "fronting" refer to the phenomenon in which the normal Gaussian peak has an asymmetry factor other than 1. Tailing is most often caused by sites on the packing that have a stronger than normal retention for the solute.

"A retention volume" (Rt) is the volume of the mobile phase required to elute a component or a certain point of the separation profile through a resin bed. The retention volume of a component may be expressed as % of the resin bed volume. In connection with the present invention, a retention volume especially refers to the volume required to elute the start of a product component fraction (such as sucrose and betaine product fractions) through the column.

"A void" or "void volume" in connection with the present invention refers to the volume required to elute the start of the conductivity peak (salts) through the column.

"BV" refers to the resin bed volume of columns, partial packed beds or a separation system.

"Peak broadening" refers to the dispersion of a chromatographic peak (separation profile) as it moves through the column.

"Volume of steps" (V) refers to the volume of the mobile phase (including the feed, eluent and circulation and optionally PARTS), which moves a component, a separation profile or parts thereof through the separation column(s) from a predetermined step in a separation sequence to another predetermined step in the same or following sequences. The volume of steps is calculated step by step by summing up the volumes of the mobile phase transferred in each step (the volume introduced into the columns in each step during the feeding, elution and/or circulation phases and during the optional part feeding phase).

"An eluent introduction position" refers to any location in the chromatographic system where eluent may be introduced.

"DS" refers to the dissolved dry substance content. Equal to "dissolved substance content".

"Purity of a component" refers to the content of the component on DS.

"Separation capacity" refers to the amount of dissolved substance (DS) in all product fractions and residual fractions taken out of the system, expressed as kg DS /volume of separation resin (m³)/hour (h). Recycle and PART(s) are not included.

"The W/F ratio" refers to the ratio of the volume of eluent water to the volume of the feed.

### DESCRIPTION OF THE INVENTION

The invention relates to a process of separating and recovering betaine and sucrose from concentrated raw juice or thick juice containing more than 85% sucrose on DS and containing also betaine and residual components in a chromatographic sequential moving bed (SMB) system, which comprises a plurality of columns containing one or more partial packed beds, wherein the columns form one or more loops, comprising

creating at least two successive separation profiles in the system by repeating a predetermined separation sequence, which contains steps comprising one or more of a feeding phase, an elution phase and a circulation phase, whereby said at least two separation profiles are simultaneously present in the system, and each separation profile comprises a sucrose subprofile, a betaine subprofile, a residual subprofile and optionally other subprofiles, whereby the betaine subprofile and the residual subprofile of successive separation profiles are overlapping,

moving said at least two separation profiles forward through the system by repeating the predetermined separation sequence, and

recovering at least one sucrose fraction, at least one betaine fraction containing also residual components and optionally one or more further fractions.

In the present invention, said concentrated raw juice and thick juice refer to a sugar beet based syrup containing more than 85%, preferably more than 90% sucrose on DS. The raw juice and thick juice contain also betaine in an amount of up to 3% on DS, and preferably in the range of 0.5% to 3% betaine on DS. Furthermore, the concentrated raw juice and thick juice contain residual components (for example salts, color compounds, organic acids, amino acids etc.) in a typical amount of less than 8% on DS. A typical example of a useful starting material in the present invention is a thick juice containing about 90% to 95% sucrose, 0.8% to 1.5% betaine and about 1% conductivity ash (salts) and 2% to 8% other inorganic/organic components.

In a preferred embodiment of the invention, two separation profiles are created in the system. In another preferred embodiment of the invention, three separation profiles are created in the system.

The chromatographic separation system of the present invention comprises a plurality of columns, which refer to more than one columns. In a typical embodiment of the invention, the system comprises three or more separation columns containing one or more partial packed beds. In a preferred embodiment of the invention, the system comprises 3 to 12 columns, preferably 6 to 12 columns.

In a typical embodiment of the invention, the columns have an equal size. The columns may also have a different size in relation to each other. The columns may also be divided into two or more compartments.

The partial packed beds of the columns are filled with a separation resin which is conventionally used for the separation of betaine and sucrose from sugar beet based solutions. Especially useful resins are strongly acid cation exchange resins (SAC resins), which may be in a monovalent or divalent form, such as in H⁺, Na⁺, K⁺ or Ca²⁺ form. A preferred ion form is a mixture of Na⁺ and K⁺. To maintain the resin in Na⁺/K⁺ form, divalent ions must be removed from the feed solution by softening.

The resins may be acrylic or styrenic resins having a crosslinking degree in the range of 1 to 20%, for example 4 to 10% DVB (divinylbenzene), preferably 4.5 to 7.5% DVB. The crosslinking degree of the resins as a rule affects the retention volume of the components. A typical mean particle size of the resins is 200 to 450 µm.

The columns/partial packed beds of the SMB system form one or more loops. In one embodiment of the invention, the system comprises a loop formed by all columns of the system in one or more steps of the separation sequence, i.e. there is a loop from the last column to the first column of the system. In a preferred embodiment of the invention, the system comprises two or three loops formed by the columns of the system at least in some of the steps of the separation sequence.

A loop may be closed or "open". In a closed loop, liquid is circulated and essentially nothing is fed or withdrawn from the loop. In an open loop, eluent or feed or PART can be introduced into the loop and a product fraction or a residual fraction or a PART or a recycle fraction can be withdrawn therefrom. During the feed, the elution and the part feeding phase, the flow through the packing material beds may take place between successive loops, wherein the flows carry material from one loop to another. During the circulation phase, the loop is closed and separated from the other loops.

The sequential SMB process of the present invention comprises sequences, steps and phases. The sequences, steps and phases are defined above. There can be 1 to 4 parallel same or different phases (selected from feeding, elution and/or circulation and optionally part feeding) in one separation step.

The fractionation is carried out using the sequences, steps and phases above, whereby a separation profile (i.e. a dry substance profile) is formed in the separation columns from the dissolved substances present in the feed. The fractionation is effected by creating the separation profile and moving the separation profile forward in the SMB system by repeating the sequence. The sequence has to be repeated at least two or three times to essentially elute the profile through the whole SMB system. In a preferred embodiment of the invention, two separation profiles are simultaneusly present in the system. In another preferred embodiment of the invention, three separation profiles are simultaneously present in the system. The separation profile comprises a sucrose subprofile, a betaine subprofile, a residual subprofile, and optionally further subprofiles.

In accordance with the process of the invention, there is overlap between the betaine subprofile and the residual subprofile of successive separation profiles. In other words, there is overlap between the betaine subprofile of one separation profile and the residual subprofile of the following separation profile. Consequently, the betaine fraction recovered is enriched in betaine and residual components from the successive separation profiles (betaine from one separation profile and residual components from the following separation profile).

In one embodiment of the invention, said at least two separation profiles are present in the same loop. In one specific embodiment of the invention, said at least two separation profiles are present in a loop formed by all columns of the system.

In a further embodiment of the invention, the process may comprise a step where the columns form two separate loops and a separation profile is moved forward in each loop, i.e. one separation profile is present in each two loops. In another embodiment of the invention, the process may comprise a step where the columns form three separate loops and a separation profile is moved forward in each loop, i.e. one separation profile is present in each three loops. For example, columns 1 and 2, columns 3 and 4 as well as columns 5 and 6 in a six-column system may form three separate loops and the separation profile is moved forward preferably simultaneously in each loop. The two-loop and three-loop arrangements facilitate advantageous color removal to the residual fractions.

Typically the first column of the system is the feed column of the system.

The feed volume is selected so that the SMB system is able to separate sucrose from betaine and salts with desired yields to provide product fractions with a desired purity. In one embodiment of the invention, the feed volume may be 6 to 15% of the total bed volume.

The feed concentration (dry substance content) is typically from 40 to 65 g dry substance/100 g feed solution and preferably from 45 to 55 g dry substance/100 g feed solution.

The eluent is typically selected from water, ion exchanged water and condensate from evaporation.

In a preferred embodiment of the invention, the amount of eluent in the elution phase is adjusted on the basis of the retention volumes of betaine and residual components to obtain overlap between the betaine subprofile and the residual subprofile of successive separation profiles.

In another preferred embodiment of the invention, the amount of eluent in the elution phase is adjusted on the basis of the retention volumes of sucrose and residual components to obtain non-overlapping of sucrose and residual components of successive separation profiles.

The retention volumes of sucrose, betaine and residual components are experimentally determined for the resin beds in use. The retention volumes of the components depend on the divinylbenzene (DVB) content of the resins, for example.

In the separation of sugar beet based syrups with strong acid cation exchange resins in a monovalent form which have a DVB content of 6 to 6.5%, the retention volume of betaine is approximately 70% (between 67 and 73%) of the resin bed volume and the retention volume of sucrose is approximately between 55% and 60% of the resin bed volume. The retention volume of the start of the conductivity peak (salts and large molecules) in the same separation with the same resins is approximately between 28 and 34% of the resin bed volume, which is equal to the void volume of the resin bed.

There is no water intervals between successive profiles at the outlet of the system and there is overlap between the successive separation profiles, contrary to US 6,875,349 B2. Especially, there is overlap between the betaine subprofile and the residual subprofile of successive separation profiles, which facilitates a higher dry substance content in the recovered fractions. This provides the advantage of considerable savings in the evaporation energy, i.e. less energy is required to concentrate the recovered fractions.

The three-profile and two-profile modes of the present invention provide, as a further advantage, short sequence times. In the three-profile mode of the present invention, the sequence time may be for example 30 minutes). In the two-profile mode of the invention, the sequence time may be for example 47 minutes. For reference, a single profile mode would take about two times 47 minutes, i.e. about 94 minutes. The sequence time can be considerably shorter, if a shorter bed length of the resin bed is in use.

Finally, the process of the invention comprises recovering at least one sucrose fraction, at least one betaine fraction containing also residual components from the following separation profile, and optionally one or more further fractions. Said further fractions may be selected from one or more residual fractions and one or more recycle fractions. Said residual fractions may be enriched in residual components of concentrated raw juice and thick juice, which residual components comprise salts as well as color compounds, organic acids, amino acids etc. Said further fractions may also comprise for example one or more recycle fractions, which are circulated back to the separation to dilute the feed.

The sucrose fraction and the betaine fraction enriched in betaine and containing also residual components may be collected from one or more selected columns of the system. In a preferred embodiment of the invention, the sucrose fraction is collected from one or more columns.

In one embodiment of the invention, at least one sucrose fraction is recovered from a column and at least one further sucrose fraction is recovered from one or more other columns of the system.

In another embodiment of the invention, said at least one betaine fraction is recovered from a column and at least one further betaine fraction is recovered from one or more other columns of the system.

Said one or more other columns are preferably selected from any upstream columns of the system in regard to the column from which said at least one sucrose/betaine fraction is recovered.

In a further embodiment of the invention, said at least one sucrose/betaine fraction and said at least one further sucrose/betaine fraction are recovered from one and the same separation profile during more than one separation sequences.

In a still further embodiment of the invention, said at least one sucrose/betaine fraction and said at least one further sucrose/betaine fraction are recovered from more than one separation profiles during one and the same separation sequence.

Said at least one sucrose fraction and said at least one betaine fraction are preferably recovered from the last column of the system.

In the process of the present invention, the last column of the system refers to the last column downstream from the feed column. The feed column is usually the first column of the system.

Surprisingly, it was found that the overlapping of the betaine subprofile and the residual subprofile of successive separation profiles in accordance with the present invention made it was possible to fit two or even three separation profiles into the SMB system. Furthermore, it was found that collecting product fractions and/or recycle fractions from several columns (several positions of the system) was also beneficial for fitting said two or three profiles in the system.

It was also surprisingly found that the fronting of the sucrose subprofile was reduced, obviously for the reason that the starting material with a high sucrose purity (>85%) already has a significantly lower salt content compared to e.g. molasses. Consequently, the sucrose subprofile and the whole separation profile remain advantageously narrow during the fractionation, which further helps to fit two and even three separation profiles into the SMB system.

Further operations to facilitate the fitting of three profiles into the SMB system and the recovery of the product fractions (especially sucrose and betaine) with a high yield and purity relates to the withdrawal of residual fractions. Residual fractions containing minor amounts of sucrose (such as 1 to 50% sucrose on DS) are withdrawn from the columns, preferably from all columns. In other words, the residual is withdrawn from the columns as soon as sucrose is sufficiently separated therefrom. Consequently, the size of the residual subprofile (salt subprofile) will decrease while the fractionation proceeds, which leads to further reduced fronting of the sucrose subprofile.

The multiprofile operation mode of the present invention provides increased separation capacity. When applying a two-profile mode instead of a one-profile mode, an increase of 100% in the separation capacity may be achieved. When applying a three-profile mode instead of a two-profile mode, an increase of 50% in the separation capacity may be achieved.

In the process of the present invention, the fractionation is effected so that the slow-moving betaine and the fast-moving residual components, such as salts of successive separation profiles are essentially recovered in the same fraction. The betaine fraction enriched in betaine and also containing salts and other residual components may be divided into several fractions, for example into a fraction further enriched in betaine and a fraction further enriched in residual components.

In a further embodiment of the invention, betaine is recovered from the betaine fraction or from said further betaine enriched fraction for example by further chromatographic fractionation, followed by crystallization.

By applying the multiprofile mode and overlapping the betaine subprofile and the residual subprofile of successive separation profiles in accordance with the present invention, betaine can be efficiently recovered from concentrated raw juice or thick juice. The yield of betaine to said at least one betaine fraction is more than 50%, preferably more than 80%, more preferably more than 85% and still more preferably more than 94%, based on the betaine of the concentrated raw juice or thick juice.

The betaine content of said at least one betaine fraction recovered from the process is more than 10% on DS, preferably more than 25% on DS, more preferably more than 30% on DS and still more preferably more than 50% on DS. In a typical embodiment of the invention, the betaine content of the betaine fraction(s) is in the range of 10% to 60%, preferably 25% to 60% on DS.The dry substance content of said at least one betaine fraction is more than 1 weight-%, preferably more than 2 weight-% and more preferably more than 3 weight-%, typically from 2 to 3.5 weight-%.

The betaine content and the dry substance content of optional further betaine fractions recovered from the process are the same as above.

The process of the invention also provides one or more sucrose fractions with improved quality, having a sucrose purity of more than 92%, preferably more than 95% and more preferably more than 98% on DS. The betaine content of the sucrose fractions is less than 0.5%, preferably less than 0.2% and more preferably less than 0.1 %, and the salt content thereof is lower than 50% of the salts in the starting material. Furthermore, the process of the invention efficiently removes conductivity ash, color and monosaccharide impurities from sucrose.

The process of the invention provides a sucrose yield of more than 98%, preferably more than 99% (based on sucrose in the thick juice or concentrated raw juice used as the starting material). The removal of the residual components to the betaine fraction and to the optional residual fraction(s) is more than 60% calculated from the content of the residual components in the starting material (thick juice or concentrated raw juice).

The process of the invention provides a high separation capacity, for example a separation capacity of about 100 kg dry substance per hour per m³ resin and a sequence time of about 47 minutes for a two-profile embodiment of the invention, and a separation capacity of more than 150 kg dry substance per hour per m³ resin and a sequence time of about 30 minutes for a three-profile embodiment of the invention.

In a specific embodiment of the invention disclosed in Example 1, the process provides a separation capacity of up to 154 kg dry substance per hour per m³ resin. Furthermore, the process provides a favourable ratio of eluent water to the feed dry substance, for example about 4.9 m³ water per ton dry substance. This corresponds to a W/F ratio (the ratio of the volume of eluent water to the volume of feed) of 3.0 when the feed has dry substance content about 50%.

Consequently, in accordance with the multi-profile process of the present invention, betaine can be enriched and recovered from a raw material having a very low betaine content (such as about 1 %) considerably more efficiently (for example with a yield of approximately 80 to 90%) than the prior art methods, which do not recover betaine at all. Furthermore, the process provides a sucrose fraction with a very low betaine content, a low salt content and a high sucrose yield. The process provides a significantly improved production capacity compared to the prior art methods. The increase of the process capacity may be in the range of 50% to 650%.

Further advantages, such as significant reduction of the amount of eluent water (from 10 up to 50%) in the separation, can be achieved by applying the methods of WO 2010/097513 A1 and WO 2010/097511 A1, which are incorporated herein by reference. The methods disclosed in these applications generally disclose the introduction of various parts of the separation profile back to the separation as eluent replacement. The reduction of the amount of eluent water leads to lower energy requirement in the evaporation of the separated fractions.

Consequently, in a further embodiment of the invention, one or more parts of said at least two separation profiles may be introduced back to one or more of eluent introduction positions of the system to substitute a portion of the eluent, wherein said parts comprise components selected from sucrose and betaine and residual components.

One example of a suitable part of the separation profile for eluent replacement comprises the overlapping part of the betaine subprofile and the residual subprofile. Furthermore, parts of said residual fractions or parts of said one or more further fractions (for example dilute salt fractions) may be circulated back to the separation as a substitute of eluent.

The generation of three separation profiles in the same loop for thick juice is illustrated in Example 1, which discloses a three-profile SMB separation of thick juice into a sucrose fraction and a betaine fraction enriched in betaine and salts. Example 2 illustrates a corresponding two profile separation.

Several advantages can be seen when comparing the multi-profile method of Example 1 with the prior art method known from US 5 466 294. First, in accordance with the multiprofile method of the invention, betaine can be enriched into the betaine fraction up to a content of approximately 15% on DS (the content of betaine in the raw material was as low as about 1 %). In accordance with the present invention, approximately 90% betaine can be recovered from thick juice into the betaine fraction. The prior art methods do not recover a betaine fraction at all from softened sucrose solutions derived sugar beet. Betaine can be easily recovered from the betaine fraction for example by chromatographic separation, followed by crystallization. As a second advantage, the method of the present invention provides a sucrose fraction with an improved sucrose purity (98.5% on DS vs. 97.4% on DS), with a very low betaine content of 0.1 % (compared to 0.9% in the prior art method), with very low salt content and with a very high sucrose yield of up to 99.5%. As a third advantage, the production capacity (calculated as kg dry substance / hour / m³ of the resin) is approximately 6.5-fold compared to the capacity of the prior art method (154 kg dry substance / h / m³ of the resin vs. approximately 23 kg dry substance / h / m³ of the resin).

Corresponding advantages can also be seen when comparing the multiprofile method of Example 1 with the prior art method known from US 6 224 683 B1. For example, the process of the invention provides a sucrose fraction with an improved sucrose purity because of multifold improvement in the removal of other organic compounds (other saccharides, betaine, amino acids etc.) from the sucrose fraction. The process of the invention also provides considerably improved production capacity (154 kg dry substance / h / m³ of the resin vs. approximately 64.2 kg dry substance / h / m³ of the resin).

### EXAMPLE 1

### MULTIPROFILE SEQUENTIAL SMB SEPARATION OF THICK JUICE

The process equipment includes 10 columns, required piping, a feed pump, recycling pumps, an eluent water pump, heat exchangers, feed and eluent and product tanks, flow control means for the out-coming liquids as well as inlet and product valves for the various process streams. The height of all columns is 1.85 m and the diameter of all columns is 3.6 m. The columns are packed with a strong acid gel type cation exchange resin (Amberlite 1320K) in Na⁺ form. The mean bead size of the resin is 0.32 mm.

Before the separation, thick juice is diluted with water and carbonated with sodium carbonate to reduce the calcium level of the solution. Finally, thick juice is pre-coat filtered using diatomaceous earth as a filtering aid.

The feed is composed as set forth below, whereby the percentages are given on a dry substance weight basis.

**TABLE E1-1**

| Composition of the feed | |
|---|---|
| Feed pH | 8.9 |
| Feed dry substance, g/100 g | 50.0 |
| Sucrose, % on DS | 93.0 |
| Betaine, % on DS | 1.0 |
| Others, % on DS | 6.0 |

The fractionation is performed by way of a 10-step SMB sequence as set forth below. A mode with three separation profiles in a loop is applied, and two very pure sucrose fractions and several betaine fractions enriched in betaine and residual components are collected. The feed and the eluent are used at a temperature of 80°C and evaporation condensate is used as an eluent.

Step 1: 4.0 m³ of feed solution is pumped into column 1 at a flow rate of 100 m³/h, and a sucrose fraction is collected from column 10.

Step 2: 4.0 m³ of feed solution is pumped into column 1 at a flow rate of 67 m³/h, and a betaine fraction is collected from the same column. Simultaneously, 4.0 m³ of eluent is pumped into column 2 at a flow rate of 67 m³/h, and a betaine fraction is collected from column 5. Also simultaneously, 6.0 m³ of eluent is pumped into column 6 at a flow rate of 100 m³/h, and a sucrose fraction is collected from column 8. Also simultaneously, 4.0 m³ of eluent is pumped into column 10 at a flow rate of 67 m³/h, and a betaine fraction is collected from column 9.

Step 3: 6.0 m³ of feed solution is pumped into column 1 at a flow rate of 100 m³/h, and a sucrose fraction is collected from column 8. Simultaneously, 6.0 m³ is looped at a flow rate of 100 m³/h in columns 9 and 10.

Step 4: 4.0 m³ of feed solution is pumped into column 1 at a flow rate of 100 m³/h, and a betaine fraction is collected from column 2. Simultaneously, 4.0 m³ of eluent is pumped into column 3 at a flow rate of 100 m³/h, and a betaine fraction is collected from column 6. Also simultaneously, 4.0 m³ of eluent is pumped into column 7 at a flow rate of 100 m³/h, and a sucrose fraction is collected from column 8. Also simultaneously, 4.0 m³ of eluent is pumped into column 9 at a flow rate of 100 m³/h, and a betaine fraction is collected from column 10.

Step 5: 2.0 m³ of feed solution is pumped into column 1 at a flow rate of 100 m³/h, and a sucrose fraction is collected from column 8. Simultaneously, 2.0 m³ is looped at a flow rate of 100 m³/h in columns 9 and 10.

Step 6: 4.0 m³ of eluent is pumped into column 1 at a flow rate of 100 m³/h, and a sucrose fraction is collected from column 10.

Step 7: 4.0 m³ of eluent is pumped into column 1 at a flow rate of 100 m³/h, and a betaine fraction is collected from column 3. Simultaneously, 4.0 m³ of eluent is pumped into column 4 at a flow rate of 100 m³/h, and a betaine fraction is collected from column 7. Also simultaneously, 4.0 m³ of eluent is pumped into column 8 at a flow rate of 100 m³/h, and a betaine fraction is collected from column 9. Also simultaneously, 4.0 m³ of eluent is pumped into column 10 at a flow rate of 100 m³/h, and a sucrose fraction is collected from the same column.

Step 8: 6.0 m³ of eluent is pumped into column 1 at a flow rate of 100 m³/h, and a sucrose fraction is collected from column 10.

Step 9: 4.0 m³ of eluent is pumped into column 1 at a flow rate of 100 m³/h, and a betaine fraction is collected from column 4. Simultaneously, 4.0 m³ of eluent is pumped into column 5 at a flow rate of 100 m³/h, and a betaine fraction is collected from column 8. Also simultaneously, 4.0 m³ of eluent is pumped into column 9 at a flow rate of 100 m³/h, and a betaine fraction is collected from column 10.

Step 10: 2.0 m³ is looped at a flow rate of 100 m³/h in columns 1, 2, 3 and 4. Simultaneously 2.0 m³ is looped at a flow rate of 100 m³/h in columns 5, 6, 7 and 8. Also simultaneously, 2.0 m³ is looped at a flow rate of 100 m³/h in columns 9 and 10.

The system and the resin ion form were balanced with 10-18 sequences, and the above mentioned betaine fractions and sucrose fractions are collected. The results including HPLC analyses for the combined fractions are set forth in the table below.

**TABLE E1-2**

| | Combined betaine | Combined sucrose |
|---|---|---|
| Volume, m³ | 48.0 | 32.0 |
| Dry substance, weiqht-% | 1.6 | 31.8 |
| Dry substance, g/100 ml | 1.6 | 36.1 |
| Sucrose, % on DS | 7.7 | 98.5 |
| Betaine, % on DS | 14.9 | 0.1 |
| Others, % on DS | 77.4 | 1.4 |

The overall yield calculated from these fractions is 99.5% for sucrose. The yield of betaine to the betaine fractions is 90%. The separation capacity for the fractionation is 154 kg dry substance per hour per m³ of resin, and the ratio of the eluent water to the feed dry substance is 4.9 m³ water per ton dry substance. This corresponds to a W/F ratio of 3.0 (vol/vol).

In the fractionation, a sucrose fraction with a clearly improved quality is obtained, and it is also possible to recover betaine with good yield and economical purity to the betaine fractions for further purification. When the results are compared to the prior art method of US 5 466 294, it can be seen that a clearly improved sucrose purity can be achieved with approximately 6.5-fold separation capacity.

In the example, only two type of product fractions are collected, i.e. sucrose fractions and betaine fractions. However, it is also possible to divide the betaine fractions separately into several portions and recover a fraction enriched in betaine and a fraction enriched in residual components separately.

### EXAMPLE 2 TWO PROFILE SEQUENTIAL SMB SEPARATION OF THICK JUICE WITH PART

The process equipment included eight columns connected in series, a feed pump, recycling pumps, an eluent water pump, heat exchangers, flow control means for the out-coming liquids as well as inlet and product valves for the various process streams. The height of all columns was 2 m and the diameter was 0.111 m. The columns were packed with a strong acid gel type cation exchange resin Amberlite CR 1320 in Na⁺-form. The mean bead size of the resin was 0.32 mm.

Before the separation, thick juice was diluted with water and carbonated with sodium carbonate to reduce the calcium level of the solution. Finally, thick juice was pre-coat filtered using diatomaceous earth as a filter aid. The feed was composed as set forth below, whereby the percentages are given on a dry substance weight basis.

**TABLE E2-1**

| Composition of thick juice | |
|---|---|
| Sucrose, % on DS | **90.7** |
| Betaine, % on DS | 0.9 |
| Trisaccharides, % on DS | 1.2 |
| Disaccharides, % on DS | 0.0 |
| Glucose, % on DS | 0.1 |
| Fructose, % on DS | 0.1 |
| Inositol, % on DS | 0.1 |
| Glycerol, % on DS | 0.1 |
| Others (mainly salts), % on DS | 6.8 |
| Feed concentration, g/100ml | 64.7 |

The fractionation was performed by way of an 11-step SMB sequence as set forth below. The aim of the separation was to separate sucrose and betaine contained therein. The feed and the eluent were used at a temperature of 80°C and ion exchanged water was used as an eluent.

Step 1: 4.1 l of feed was pumped to column 1 at flow rate of 50 l/h, and a sucrose fraction was taken from column 8 (the last column).

Step 2: 7.5 l of water was pumped to column 6 at a flow rate of 86 l/h, and a sucrose fraction was collected from the last column.

Step 3: 3.5 l of feed was pumped to column 1 at flow rate of 60 l/h, and a sucrose fraction was collected from last column.

Step 4: 6.2 l of feed was pumped to column 1 at flow rate of 78 l/h, and a sucrose fraction was collected from column 4. Simultaneously, 6.2 l was circulated in the column loop, formed with columns 5, 6, 7 and 8 at a flow rate of 78 l/h.

Step 5: 3.2 l of feed was pumped to column 1 at a flow rate of 40 l/h and a residual fraction was collected from column 2. Simultaneously, 7.0 l water was pumped to column 3 at a flow rate of 85 l/h, and a sucrose fraction was collected from column 4. Also simultaneously, 1.3 l of water was pumped to column 7 at a flow rate of 16 l/h, and a residual fraction was collected from column 6.

Step 6: 1.7 l of feed was pumped to column 1 at flow rate of 45 l/h and a sucrose fraction was collected from the last column.

Step 7: 4.5 l of PART (collected from column 8) was pumped to column 1 from an external tank at a flow rate of 42 l/h, and a sucrose fraction was collected from last column.

Step 8: 3.5 l of water was pumped to column 1 at a flow rate of 66 l/h, and a residual fraction was collected from column 3. Simultaneously, 4.5 l of water was pumped to column 4 at a flow rate of 85 l/h, and a PART fraction was collected from column 8 to an external tank.

Step 9: 6.2 l of water was pumped to column 1 at a flow rate of 65 l/h, and a betaine fraction was collected from column 8.

Step 10: 3.5 l of water was pumped to column 1 at a flow rate of 80 l/h, and a betaine was collected from column 4. Simultaneously, 3.5 l of water was pumped to column 5 at a flow rate of 80 l/h, and a betaine fraction was collected from column 8.

Step 11: 2.1 l was circulated in the column loop, formed with all columns, at a flow rate of 24 l/h.

The system and the resin the ion form were balanced with 10-18 sequences and the following fractions were drawn from the system: a residual fraction from columns 2, 3 and 6, sucrose product fractions from columns 4 and 8 and betaine product fractions from columns 4 and 8. The results including HPLC analyses for the combined fractions are set forth in Table E2-2. Table E2-3 shows separation results calculated from step volumes, column dimensions and HPLC results.

**TABLE E2-2**

| | Combined residual | Sucrose Column 4 | Sucrose Column 8 | Combined sucrose | Combined betaine |
|---|---|---|---|---|---|
| Volume, I | 8.0 | 13.2 | 21.3 | 34.5 | 13.2 |
| Concentration, g/100ml | 3.4 | 49.4 | 23.5 | 33.4 | 2.8 |
| Sucrose % on DS | 38.1 | 95.5 | 96.3 | 95.8 | 8.4 |
| Betaine, % on DS | 0.3 | 0.1 | 0.2 | 0.2 | 24.9 |
| Others, % on DS | 61.6 | 4.4 | 3.5 | 4.0 | 66.7 |

**TABLE E2-3**

| | |
|---|---|
| Separation parameters | |
| Sucrose yield, % | 98.8 |
| Betaine yield, % | 83.4 |
| Feed load, kq DS/m³ | 79.0 |
| Water/feed ratio (vol/vol) | 2.0 |
| Sequence time, min | 48.7 |
| Product capacity, kg DS/m³h | 97.3 |

Calculation of where reintroduced PART dry substance ended up was done assuming that the dry substance has a void of 32% bed volume since in this case PART is mainly composed of salts. Also band broadening was taken into account empirically. The introduced PART movement was calculated by subtracting the volume of a step from the column void starting with the step were eluent was replaced by eluent replacement solution (PART). This method was very accurate for the PART profile front movement.

## Claims

1. A process of separating and recovering betaine and sucrose from concentrated raw juice or thick juice containing more than 85% sucrose on DS and containing also betaine and residual components in a chromatographic sequential moving bed (SMB) system, which comprises a plurality of columns containing one or more partial packed beds, wherein the columns form one or more loops, comprising
creating at least two successive separation profiles in the system by repeating a predetermined separation sequence, which contains steps comprising one or more of a feeding phase, an elution phase and a circulation phase, whereby said at least two separation profiles are simultaneously present in the system, and each separation profile comprises a sucrose subprofile, a betaine subprofile, a residual subprofile and optionally other subprofiles, whereby the betaine subprofile and the residual subprofile of successive separation profiles are overlapping,
moving said at least two separation profiles forward through the system by repeating the predetermined separation sequence, and
recovering at least one sucrose fraction, at least one betaine fraction containing also residual components and optionally one or more further fractions.

2. A process as claimed in claim 1, wherein the amount of eluent in the elution phase is adjusted on the basis of the retention volumes of betaine and residual components to obtain overlap between the betaine subprofile and the residual subprofile of successive separation profiles.

3. A process as claimed in claim 1, wherein the amount of eluent in the elution phase is adjusted on the basis of the retention volumes of sucrose and residual components to obtain non-overlapping of sucrose and residual components of successive separation profiles.

4. A process as claimed in claim 1, wherein two or three-separation profiles are created in the system.

5. A process as claimed in claim 1, wherein at least one sucrose fraction is recovered from a column and at least one further sucrose fraction is recovered from one or more other columns of the system.

6. A process as claimed in claim 1, wherein at least one betaine fraction is recovered from a column and at least one further betaine fraction is recovered from one or more other columns of the system.

7. A process as claimed in claim 5 or 6, wherein said one or more other columns are selected from any upstream columns of the system in regard to the column from which said at least one sucrose/betaine fraction is recovered.

8. A process as claimed in claim 5 or 6, wherein said at least one sucrose/betaine fraction and said at least one further sucrose/betaine fraction are recovered from one and the same separation profile during more than one separation sequences or
from more than one separation profiles during one and the same separation sequence.

9. A process as claimed in claim 5 or 6, wherein said at least one sucrose fraction and/or said at least one betaine fraction are recovered from the last column.

10. A process as claimed in claim 1, wherein said at least two separation profiles are present in a loop formed by all columns of the system.

11. A process as claimed in claim 1, wherein said plurality of columns form three separate loops and a separation profile is simultaneously moved forward in each loop.

12. A process as claimed in claim 1, wherein said plurality of columns form two separate loops and a separation profile is simultaneously moved forward in each loop.

13. A process as claimed in claim 1, wherein more than 50%, preferably more than 80%, more preferably more than 85% and still more preferably more than 94% of the betaine of the concentrated raw juice or thick juice is recovered in said at least one betaine fraction,
wherein the betaine content of said at least one betaine fraction is more than 10%, preferably more than 25%, more preferably more than 30% and most preferably more than 50% on the dry substance (DS), and
wherein the dry substance content of said at least one betaine fraction is more than 1 weight-%, preferably more than 2 weight-% and more preferably more than 3 weight-%.

14. A process as claimed in claim 1, wherein the process provides a sucrose yield of more than 98%, preferably more than 99% based on the sucrose in the concentrated raw juice or thick juice and
wherein the sucrose content of said at least one sucrose fraction is more than 92%, preferably more than 95% and more preferably more than 98% on DS.

15. A process as claimed in claim 1, wherein the process further comprises introducing one or more parts of said at least two separation profiles back to one or more of eluent introduction positions of the system to substitute a portion of the eluent, wherein said parts comprise components selected from sucrose and betaine and residual components.

## Patentansprüche

1. Prozess zur Trennung und Rückgewinnung von Betain und Saccharose aus konzentriertem Rohsaft oder Dicksaft, der mehr als 85% Saccharose in TS enthält und auch Betain und Rückstandskomponenten enthält, in einem chromatographischen sequentiellen beweglichen Bettsystem (SMB), das eine Vielzahl von Säulen aufweist, die ein oder mehr Teilschüttbette enthalten, wobei die Säulen eine oder mehr Schleifen ausbilden, bei dem
zumindest zwei aufeinander folgende Trennprofile im System erzeugt werden, indem eine vorbestimmte Trennsequenz wiederholt wird, die Schritte umfasst, die eine oder mehr von einer Zuführungsphase, einer Elutionsphase und einer Zirkulationsphase aufweisen, wobei die besagten zumindest zwei Trennprofile gleichzeitig im System vorliegen und jedes Trennprofil ein Saccharose-Teilprofil, ein Betain-Teilprofil, ein Rückstand-Teilprofil und wahlweise andere Teilprofile aufweist, wobei sich das Betain-Teilprofil und das Rückstand-Teilprofil von aufeinander folgenden Trennprofilen überlappen,
die besagten zumindest zwei Trennprofile vorwärts durch das System bewegt werden, indem die vorbestimmte Trennsequenz wiederholt wird, und
zumindest eine Saccharosefraktion, zumindest eine Betainfraktion, die auch Rückstandskomponenten enthält, und wahlweise eine oder mehr weitere Fraktionen rückgewonnen werden.

2. Prozess nach Patentanspruch 1, wobei die Eluentmenge in der Elutionsphase aufgrund der Retentionsvolumen von Betain und Rückstandskomponenten eingestellt wird, um eine Überlappung zwischen dem Betain-Teilprofil und dem Rückstand-Teilprofil von aufeinander folgenden Trennprofilen zu erreichen.

3. Prozess nach Patentanspruch 1, wobei die Eluentmenge in der Elutionsphase aufgrund der Retentionsvolumen von Saccharose und Rückstandskomponenten eingestellt wird, um eine Nicht-Überlappung von Sachharose und Rückstandskomponenten von aufeinander folgenden Trennprofilen zu erreichen.

4. Prozess nach Patentanspruch 1, wobei zwei oder drei Trennprofile im System erzeugt werden.

5. Prozess nach Patentanspruch 1, wobei zumindest eine Saccharosefraktion aus einer Säule rückgewonnen wird und zumindest eine weitere Saccharosefraktion aus einer oder mehr anderen Säulen des Systems rückgewonnen wird.

6. Prozess nach Patentanspruch 1, wobei zumindest eine Betainfraktion aus einer Säule rückgewonnen wird und zumindest eine weitere Betainfraktion aus einer oder mehr anderen Säulen des Systems rückgewonnen wird.

7. Prozess nach Patentanspruch 5 oder 6, wobei die besagten eine oder mehr anderen Säulen aus beliebigen Säulen des Systems ausgewählt werden, die sich flussaufwärts in Bezug auf die Säule, aus der die besagte zumindest eine Saccharose-/Betainfraktion rückgewonnen wird, befinden.

8. Prozess nach Patentanspruch 5 oder 6, wobei die besagte zumindest eine Saccharose-/Betainfraktion und die besagte zumindest eine weitere Saccharose-/Betainfraktion aus demselben Trennprofil während mehr als einer Trennsequenz oder
aus mehr als einem Trennprofil während derselben Trennsequenz rückgewonnen werden.

9. Prozess nach Patentanspruch 5 oder 6, wobei die besagte zumindest eine Saccharosefraktion und/oder die besagte zumindest eine Betainfraktion aus der letzten Säule rückgewonnen werden.

10. Prozess nach Patentanspruch 1, wobei die besagten zumindest zwei Trennprofile in einer von allen Säulen des Systems ausgebildeten Schleife vorliegen.

11. Prozess nach Patentanspruch 1, wobei die besagte Vielzahl von Säulen drei separate Schleifen ausbilden und ein Trennprofil gleichzeitig in jeder Schleife vorwärts bewegt wird.

12. Prozess nach Patentanspruch 1, wobei die besagte Vielzahl von Säulen zwei separate Schleifen ausbilden und ein Trennprofil gleichzeitig in jeder Schleife vorwärts bewegt wird.

13. Prozess nach Patentanspruch 1, wobei mehr als 50%, vorteilhaft mehr als 80%, vorteilhafter mehr als 85% und noch vorteilhafter mehr als 94% des Betains des konzentrierten Rohsafts oder Dicksafts in der besagten zumindest einen Betainfraktion rückgewonnen werden,
wobei der Betaingehalt der besagten zumindest einen Betainfraktion mehr als 10%, vorteilhaft mehr als 25%, vorteilhafter mehr als 30% und am vorteilhaftesten mehr als 50% in Trockensubstanz (DS) beträgt und
wobei der Trockensubstanzgehalt der besagten zumindest einen Betainfraktion mehr als 1 Gew.-%, vorteilhaft mehr als 2 Gew.-% und vorteilhafter mehr als 3 Gew.-% beträgt.

14. Prozess nach Patentanspruch 1, wobei der Prozess eine Saccharoseausbeute von mehr als 98%, vorteilhaft mehr als 99%, bezogen auf die Saccharose im konzentrierten Rohsaft oder Dicksaft, bereitstellt und
wobei der Saccharosegehalt der besagten zumindest einen Saccharosefraktion mehr als 92%, vorteilhaft mehr als 95% und vorteilhafter mehr als 98% in TS beträgt.

15. Prozess nach Patentanspruch 1, wobei der Prozess ferner aufweist: Einführung von einem oder mehr Teilen der besagten zumindest zwei Trennprofile zurück in eine oder mehr von Eluent-Einführungspositionen des Systems, um einen Anteil des Eluents zu ersetzen, wobei die besagten Teile Komponenten aufweisen, die aus Saccharose und Betain und Rückstandskomponenten ausgewählt sind.

## Revendications

1. Procédé de séparation et de récupération de bétaïne et de saccharose d'un jus brut concentré ou d'un jus épais contenant plus de 85 % de saccharose par rapport à la matière sèche et contenant également de la bétaïne et des composants résiduels dans un système chromatographique séquentiel à lit mobile (SMB), qui comprend une pluralité de colonnes contenant au moins un lit à garnissage partiel, dans lequel les colonnes forment une ou plusieurs boucles, comprenant
la création d'au moins deux profils de séparation successifs dans le système par répétition d'une séquence de séparation prédéterminée, qui contient les étapes comprenant au moins l'une d'une phase d'alimentation, d'une phase d'élution et d'une phase de circulation, lesdits au moins deux profils de séparation étant simultanément présents dans le système, et chaque profil de séparation comprend un sous-profil saccharose, un sous-profil bétaïne, un sous-profil résiduel et facultativement d'autres sous-profils, le sous-profil bétaïne et le sous-profil résiduel de profils de séparation successifs se chevauchant,
le déplacement desdits au moins deux profils de séparation vers l'avant dans le système par répétition de la séquence de séparation prédéterminée, et
la récupération d'au moins une fraction de saccharose, d'au moins une fraction de bétaïne contenant également des composants résiduels et facultativement d'au moins une fraction supplémentaire.

2. Procédé selon la revendication 1, dans lequel la quantité d'éluant dans la phase d'élution est ajustée sur la base des volumes de rétention de la bétaïne et des composants résiduels pour obtenir un chevauchement entre le sous-profil de la bétaïne et le sous-profil résiduel de profils de séparation successifs.

3. Procédé selon la revendication 1, dans lequel la quantité d'éluant dans la phase d'élution est ajustée sur la base des volumes de rétention du saccharose et des composants résiduels pour obtenir un non-chevauchement du saccharose et des composants résiduels de profils de séparation successifs.

4. Procédé selon la revendication 1, dans lequel deux ou trois profils de séparation sont créés dans le système.

5. Procédé selon la revendication 1, dans lequel au moins une fraction de saccharose est récupérée d'une colonne et au moins une fraction de saccharose supplémentaire est récupérée d'une ou de plusieurs autres colonnes du système.

6. Procédé selon la revendication 1, dans lequel au moins une fraction de bétaïne est récupérée d'une colonne et au moins une fraction de bétaïne supplémentaire est récupérée d'une ou plusieurs autres colonnes du système.

7. Procédé selon la revendication 5 ou la revendication 6, dans lequel ladite ou lesdites autres colonnes sont sélectionnées parmi toute colonne du système en amont relativement à la colonne de laquelle ladite ou lesdites fractions de saccharose/bétaïne sont récupérées.

8. Procédé selon la revendication 5 ou la revendication 6, dans lequel ladite ou lesdites fractions de saccharose/bétaïne et ladite ou lesdites fractions de saccharose/bétaïne supplémentaires sont récupérées d'un seul et même profil de séparation durant plus d'une séquence de séparation ou
de plus d'un profil de séparation durant une seule et même séquence de séparation.

9. Procédé selon la revendication 5 ou la revendication 6, dans lequel ladite ou lesdites fractions de saccharose et/ou ladite ou lesdites fractions de bétaïne sont récupérées de la dernière colonne.

10. Procédé selon la revendication 1, dans lequel lesdits au moins deux profils de séparation sont présents dans une boucle formée par toutes les colonnes du système.

11. Procédé selon la revendication 1, dans lequel les colonnes de ladite pluralité de colonnes forment trois boucles distinctes et un profil de séparation se déplace simultanément vers l'avant dans chaque boucle.

12. Procédé selon la revendication 1, dans lequel les colonnes de ladite pluralité de colonnes forment deux boucles distinctes et un profil de séparation se déplace simultanément vers l'avant dans chaque boucle.

13. Procédé selon la revendication 1, dans lequel plus de 50 %, de préférence plus de 80 %, de manière davantage préférée plus de 85 % et de manière encore davantage préférée plus de 94 % de la bétaïne du jus brut concentré ou du jus épais sont récupérés de ladite ou desdites fractions de bétaïne,
dans lequel la teneur en bétaïne de ladite ou desdites fractions de bétaïne est supérieure à 10 %, de préférence supérieure à 25 %, de manière d'avantage préférée supérieure à 30 % et idéalement supérieure à 50 % par rapport à la matière sèche (DS), et
dans lequel la teneur en matière sèche de ladite ou desdites fractions de bétaïne est supérieure à 1 % en poids, de préférence supérieure à 2 % en poids et de manière davantage préférée supérieure à 3 % en poids.

14. Procédé selon la revendication 1, dans lequel le procédé fournit un rendement en saccharose supérieur à 98 %, de préférence supérieur à 99 % sur la base du saccharose dans le jus brut concentré ou dans le jus épais, et
dans lequel la teneur en saccharose de ladite ou desdites fractions de saccharose est supérieure à 92 %, de préférence supérieure à 95 % et idéalement supérieure à 98 % par rapport à la matière sèche.

15. Procédé selon la revendication 1, dans lequel le procédé comprend en outre la réintroduction d'une ou de plusieurs parties desdits au moins deux profils de séparation à une ou plusieurs positions d'introduction d'éluant du système pour remplacer une partie de l'éluant, dans lequel lesdites parties comprennent des composants sélectionnés parmi le saccharose et la bétaïne et les composants résiduels.
